# EUROPÄISCHE PATENTSCHRIFT

(11) **EP 0 674 877 B1**
(45) Veröffentlichungstag und Bekanntmachung des Hinweises auf die Patenterteilung: **16.08.2001**
(21) Anmeldenummer: 95250076.7
(22) Anmeldetag: 28.03.1995
(51) Int. Cl.: A61B 17/12

(54) **Medizinisches Instrument zum gezielten Setzen eines Knotens**
Surgical instrument for the precise placement of a knot
Instrument médical destiné au placement ciblé d'un noeud

(30) Priorität: 31.03.1994 DE 4411827
(43) Veröffentlichungstag der Anmeldung: 04.10.1995
(73) Patentinhaber: Otten, Gert, Prof., Dr., 27619 Schiffdorf (DE)
(72) Erfinder: Otten, Gert, Prof. Dr. med., D-27619 Schiffdorf (DE); Lindeke, Carsten, D-12459 Berlin (DE)
(74) Vertreter: Porth, Hans-Joachim

(56) Entgegenhaltungen:
- WO-A-94/08515
- GB-A- 2 247 841
- US-A- 2 433 956
- US-A- 2 595 086
- US-A- 3 834 395

## Beschreibung

Die Erfindung betrifft ein medizinisches Instrument nach dem Oberbegriff des Patentanspruchs 1.

In der Medizin werden Gewebe, Organe und dgl. häufig mittels chirurgischen Garns verbunden, indem das Garn durch das Gewebe hindurch oder um das Organ herum zu einer Schlaufe angeordnet und die Schlaufe sodann mittels eines Knotens zusammengezogen wird, bis das Gewebe bzw. Organ die gewünschte Position inne haben. Meist wird anschließend zur Sicherung dieser Position ein weiterer Knoten auf den ersten Knoten aufgesetzt. Das Setzen der Knoten ist insbesondere dann sehr schwierig, wenn die Nahtstelle schlecht zugänglich ist. Besonders problematisch ist das Setzen des Knotens beim Eingriff in einen geschlossenen Körper. Hierbei müssen, nachdem die Schlaufe durch das Gewebe bzw. Organ gelegt ist, die beiden freien Enden über einen Trokar aus dem Körper heraus geführt und sodann verknotet werden. Anschließend wird der Knoten mittels eines Instruments durch den Trokar zur Nahtstelle verschoben, während die freien Enden des Gewebes außerhalb des Trokars verbleiben. Bekannte Instrumente zum Schieben des Knotens weisen einen langen Schaft auf, an dessen einem Ende eine Aufnahme oder dergleichen für den Knoten angeordnet ist. Die Aufnahme wird dem Knoten zwischen den beiden freien Enden des Garns zugeführt, bis der Knoten von ihr aufgenommen oder in sonstiger Weise gehalten ist. Nachfolgend wird er zuerst innerhalb des Trokars und anschließend innerhalb der Körperhöhle bis zur Nahtstelle verschoben. Eine exakte Handhabung des Knotens wie auch der freien Garnenden ist nicht immer gegeben. Mitunter können der Knoten oder die Garnenden von dem Instrument nicht sicher gehalten werden und gehen verloren, so daß das Knoten der Enden und Verschieben des Knotens wiederholt werden muß. Ein besonderer Nachteil ist darin zu sehen, daß der Knoten bestenfalls verschoben, nicht aber exakt gesetzt bzw. festgezogen werden kann.

Ein derartiges Instrument ist aus der US-A-2 433 956 bekannt. Hierbei ist der Instrumentenkopf im wesentlichen durch einen Hebelmechanismus aus zwei zueinander schwenkbeweglichen Hebelarmen gebildet, wobei die Hebelarme zum Setzen und Festziehen des Knotens zueinander gespreizt werden. Nachteilig dabei ist, daß die Hebelarme auch im ungespreizten Zustand wesentlich über den Umfang eines zylindrischen Grundkörpers bzw. einer zylindrischen Führungshülse vorstehen, so daß dieses Instrument schlecht in Verbindung mit einem Trokar und damit für einen Eingriff in einem geschlossenen Körper einsetzbar ist.

Ein ähnliches, allerdings sehr einfaches Instrument ist auch aus der US-A-3 834 395 bekannt, bei dem zwei zueinander schwenkbewegliche Hebel eines Instrumentenkopfs zum Festziehen eines Knotens ebenfalls zueinander spreizbar sind. Auch hierbei stehen die Hebelenden auch in ungespreiztem Zustand wesentlich über den Umfang eines Grundgörpers hervor. Der Einsatz dieses Instruments in Verbindung mit einem Trokar erscheint ausgeschlossen.

Ferner ist aus der US-A-2 595 086 ein derartiges Instrument bekannt, das einen länglichen Grundkörper und an einem Ende desselben einen starren Instrumentenkopf aufweist. Letzterer besteht im wesentlichen aus einem gegenüberliegend zum Grundkörper unterbrochenen Ring mit einer umfänglich verlaufenden Nut, in der die Garnenden zum Setzen des Knotens gehalten und geführt werden. Das exakte Setzen eines Knotens insbesondere an schlecht zugänglichen Stellen wie auch der Einsatz in Verbindung mit einem Trokar erscheint kaum möglich.

Schließlich ist aus der GB-A-2 247 841 ein derartiges Instrument bekannt, daß einen starren und im wesentlichen einteiligen Instrumentenkopf mit im wesentlichen zylindrischer Gestalt aufweist. Der Instrumentenkopf ist mit Bohrungen, Nuten und dgl. zur Führung des Garns versehen. Ein exaktes Setzen eines Knotens erscheint sehr schwierig.

Der Erfindung liegt die Aufgabe zugrunde, ein medizinisches Instrument der Eingangs erwähnten Art zu schaffen, daß in einfacher Weise eine sichere Handhabung des Knotens und der Garnenden sowie ein exaktes Setzen eines Knotens bei schlecht zugänglichen Nahtstellen ermöglicht.

Erfindungsgemäß wird die Aufgabe dadurch gelöst, daß der Instrumentenkopf eine Profilierung mit mindestens einem peripheren Bereich und einem zentralen Bereich zur separaten Aufnahme, Führung und/oder Handhabung der freien Enden des Garns und/oder Knotens und benachbarter Abschnitte der Schlaufe aufweist. Beide Bereiche sind zudem über eine Ausnehmung miteinander verbunden, in die eine dem zentralen Bereich zugeordnete koaxiale Bohrung einmündet. Damit ist gewährleistet, daß der Knoten und die benachbarten Garnbereiche sicher gehalten, in die Nähe eines Zielpunkts geschoben und dann durch Abspreizen wenigstens eines freien Endes des Garns exakt im Zielpunkt gesetzt, d.h. festgezogen werden kann. Hierdurch können sehr präzise feste Knoten auch an äußerst schlecht zugänglichen Stellen mit geringem Aufwand sicher gesetzt werden.

Der periphere Bereich der Profilierung des Instrumentenkopfs dient der Aufnahme der freien Enden des Garns während der zentrale Bereich der Aufnahme des Knotens und der angrenzenden Schlaufenbereiche dient. Beide Bereiche sind über eine Ausnehmung miteinander verbunden. Der periphere Bereich gestattet die getrennte Handhabung der freien Enden des Garns und damit eine exakte Führung des Knotens auf dem Instrumentenkopf. Die Ausnehmung dagegen ermöglicht es, den im peripheren Bereich abgleitenden Knoten übergangslos in den zentralen Bereich zu überführen.

Die Ausnehmung durchdringt den Instrumentenkopf etwa senkrecht zu dessen Mittellinie und hat vorzugsweise einen spitzwinkligen Querschnitt, so daß gezielt und separiert durch einen endlosen Schenkel der Ausnehmung der Knoten und den anderen endlichen Schenkeln getrennt jeweils eines der freien Enden des Garns geführt werden können.

Gemäß einer speziellen Ausbildung ist vorgesehen, daß die Hälften scherenförmig ausgebildet und um eine Achse schwenkbeweglich sind. Dadurch können die freien Enden derart zueinander gespreizt werden, daß der Knoten zum Zielpunkt wandern und dort festgezogen, gewissermaßen gesetzt werden kann.

Weitere Merkmale der Erfindung und deren Vorteile ergeben sich aus den übrigen Patentansprüchen und der Beschreibung.

Die Erfindung soll nachfolgend an einem Ausführungsbeispiel näher erläutert werden. In der zugehörigen Zeichnung zeigen in teilweiser und schematischer Darstellung:
- Fig. 1: die Draufsicht eines Instruments zum Setzen eines Knotens,
- Fig. 2: eine Vorderansicht eines Instrumentenkopfs, vergrößert dargestellt,
- Fig. 3: die Seitenansicht des Instrumentenkopfs nach Fig. 2,
- Fig. 4: die Ansicht einer Innenfläche einer Hälfte des Instrumentenkopfs, vergrößert dargestellt,
- Fig. 5: bis 7 die Draufsicht gemäß Fig. 1 in drei unterschiedlichen Positionen des Knotens zum Instrumentenkopf und
- Fig. 8: die Draufsicht nach Fig. 1 mit gespreizten Hälften des Instrumentenkopfs.

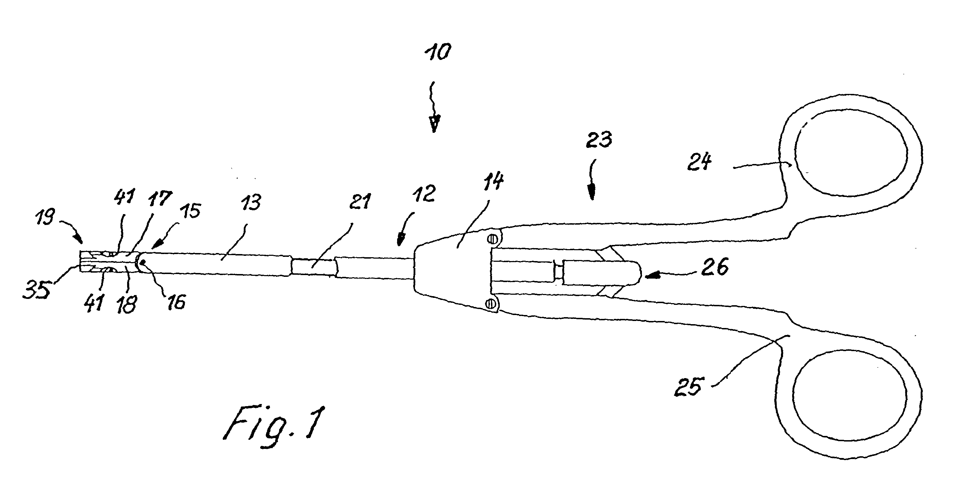

Ein medizinisches Instrument 10 (Fig.1) zum gezielten Setzen eines Knotens weist einen Grundkörper 12 auf, wobei eine Seite des Grundkörpers 12 durch eine Führungshülse 13 und die andere Seite durch eine Halterung 14 gebildet sind.

An einem Endbereich 15 der Führungshülse 13 ist eine Achse 16 angeordnet, um die zwei Hälften 17, 18 eines geteilten Instrumentenkopfs 19 zueinander schwenkbeweglich gehalten sind. Die Schwenkbewegung erfolgt mittels einer Betätigungseinrichtung, deren Schubstange 21 in der Führungshülse 13 axialverschieblich gehalten ist. Ein nicht dargestellter Endabschnitt bzw. eine nicht dargestellte Seite der Schubstange 21 ist über jeweils einen Hebel 22 gewissermaßen scherengitterartig mit jeder der Hälften 17, 18 des Instrumentenkopfs 19 verbunden, so daß die Schwenkbewegung der Hälften 17, 18 durch Axialverschiebung der Schubstange 21 innerhalb der Führungshülse 13 gegeben ist.

Der Halterung 14 des Grundkörpers 12 ist ein scherenartig ausgebildetes Grifforgan 23 mit zwei zueinander schwenkbeweglichen Griffhälften 24, 25 zugeordnet, welches zum einen zur Handhabung des gesamten Instruments 10 und zum anderen der Axialverschiebung der Schubstange 21 und damit der Schwenkbewegung der Hälften 17, 18 des Instrumentenkopfs 19 dient. Zur Axialverschiebung der Schubstange 21 ist den Griffhälften 24, 25 ein scherengitterartiger Trieb 26 zugeordnet, der mit der Schubstange 21 derart verbunden ist, daß eine Schwenkbewegung der Griffhälften 24, 25 eine Axialverschiebung der Schubstange 21 bewirkt.

Der Instrumentenkopf 19 weist eine im wesentlichen zylindrische Gestalt auf. Sein Durchmesser entspricht etwa dem der Führungshülse 13 und ist so bemessen, daß der Instrumentenkopf 19 und die Führungshülse 13 in einem Trokar verschiebbar sind und in einem inneren Spalt zwischen dem Trokar und der Führungshülse 13 zwei freie Enden 27 eines chirurgischen Garns noch Platz haben.

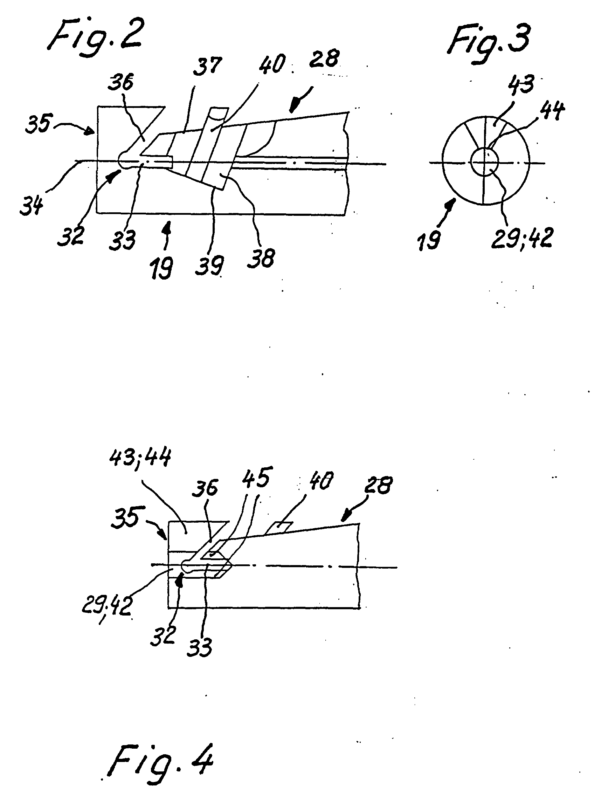

Der Instrumentenkopf 19 ist mit einer Profilierung ( Fig.2) versehen, die einen peripheren Bereich 28 zur Aufnahme, Führung und Handhabung insbesondere der freien Enden 27 des Garns und einen zentralen Bereich 29 zur Aufnahme eines von den beiden freien Enden 27 unter Ausbildung einer endlosen Schlaufe 30 geschlagenen Knotens 31 aufweist. Der periphere und zentrale Bereich 28, 29 sind über eine Ausnehmung 32 miteinander verbunden, die den Instrumentenkopf 19 etwa senkrecht zu dessen Mittellinie 34 durchdringt. Die Ausnehmung 32 hat die Form bzw. den Querschnitt eines spitzen Winkels. Ein endlicher Schenkel 33 erstreckt sich parallel zu der Mittellinie 34 des Instrumentenkopfs 19 und etwa in deren Ebene bis zu einem Stirnteil 35 des Instrumentenkopfs 19. Die lichte Weite dieses Schenkels 33 ist so bemessen, daß ein freies Ende 27 des Garns aufgenommen und geführt werden kann.

Ein endloser Schenkel 36 der Ausnehmung 32 erstreckt sich spitzwinklig von einem stirnteilseitigen Endbereich des Schenkels 33 und entgegengesetzt zum Stirnteil 35 und tritt aus dem Instrumentenkopf 19 aus. Dieser Schenkel 36 weist eine lichte Weite auf, die das Passieren des Knotens 31 gestattet.

Der periphere Bereich 28 der Profilierung ist mit einer Schräge 37 versehen, die sich vom Außenumfang des Instrumentenkopfs 19 in Richtung des Stirnteils 35 und geneigt zur Mittellinie 34 erstreckt. Die Schräge 37 erfaßt umfänglich etwa den halben Instrumentenkopf 19 und ist gleichmäßig gewölbt. Sie endet stirnteilseitig etwa dort, wo die Schenkel 33, 36 ineinander übergehen, in der Ausnehmung 32.

Der Schräge 37 sind zwischen ihrem Ausgang am Umfang des Instrumentenkopfs 19 und der Stelle, an der sie in der Ausnehmung 32 endet, etwa symmetrisch gegenüberliegend jeweils ein Freischliff 38 mit kreisabschnittförmigem Querschnitt zugeordnet, die im Bereich ihres Bodens 39 in den endlichen Schenkel 33 der Ausnehmung 32 einmünden. Der Freischliff 38 erstreckt sich jeweils vom Boden 39 zur Mittellinie 34 leicht geneigt und von dem Stirnteil 35 wegweisend. Von dem Boden 39 erstreckt sich wiederum jeweils ein zum Freischliff 38 parallel verlaufender Stift 40, so daß zwischen dem Stift 40 und dem Freischliff 38 ein nach oben offener schmaler Schlitz 41 gebildet ist. Der Stift 40 hat eine Länge, derart, daß er sich jeweils über den Freischliff 38 und die Schräge 37 erstreckt. Sie ist jedoch so bemessen, daß die Stifte 40 den Instrumentenkopf 19 in dessen radialer Richtung nicht überragen, so daß letzterer ungehindert in einem Trokar axialverschieblich ist.

Der zentrale Bereich 29 der Profilierung ist durch eine zum Instrumentenkopf 19 koaxiale Bohrung 42 gebildet, die in die Ausnehmung 32 einmündet und mittig den Schenkel 33 vollständig durchdringt (Fig.4). Der Durchmesser der Bohrung 42 ist größer als die lichte Weite des Schenkels 33, so daß die Bohrung 42 auch im Bereich des Schenkels 33 eine Seitenführung 45 aufweist, mittels der der Knoten 31 sicher in der Bohrung 42 gehalten ist. Der Durchmesser der Bohrung 42 ist ferner so bemessen, daß er etwas größer als die Erstreckung des Knotens 31 in radialer Richtung des Instrumentenkopfs 19 ist.

Schließlich ist der Bohrung 42 ein Längsschlitz 43 zugeordnet, der sich im Stirnteil 35 parallel zur Mittellinie 34 und symmetrisch zu der Schräge 37 sowie auf der Seite der letzteren erstreckt. Der Längsschlitz 43 ist keilförmig ausgebildet und verjüngt sich vom Umfang des Stirnteils 35 zu einem schmalen Spalt 44 und geht mit diesem Spalt 44 in die Bohrung 42 über. Der Längsschlitz 43 ist in Längsrichtung des Instrumentenkopfs 19 zudem zur Stirnfläche des Stirnteils 35 hin konisch aufgeweitet, wobei der Längsschlitz 43 und Spalt 44 wie auch der periphere und zentrale Bereich 28, 29 der Profilierung den beiden Hälften 17, 18 des Instrumentenkopfs 19 symmetrisch zugeordnet sind.

Das Setzen des Knotens 31 mittels des Instruments 10 erfolgt in nachstehend beschriebener Weise.

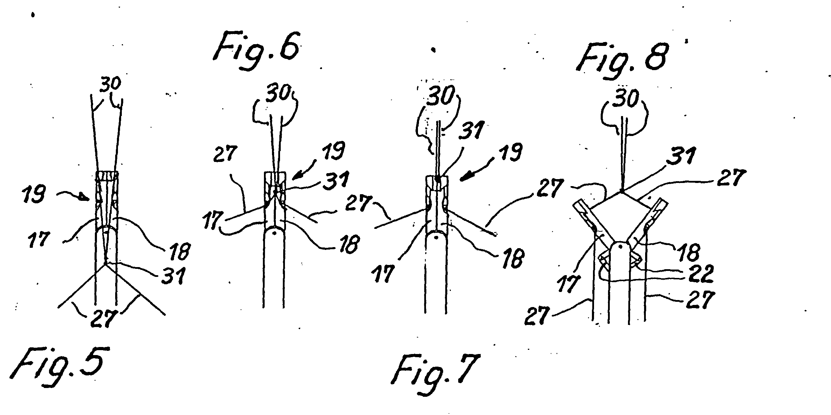

Nachdem mittels der freien Enden 27 des Garns der Knoten 31 unter Ausbildung einer durch das Gewebe oder um ein Organ verlaufenden Schlaufe 30 verknotet wurden, wird das Instrument 10 zum Knoten 31 derart positioniert, daß der Knoten 31 auf der Führungshülse 13 etwa mittig aufliegt. Die beiden freien Enden 27 sind dabei seitlich etwas um die Führungshülse 13 herum und zueinander gespreizt gehalten (Fig.5). Dabei werden die freien Enden unter leichtem Zug gehalten, so daß das Garn insgesamt gestrafft angeordnet ist. Nachfolgend wird das Instrument 10 so geführt, daß der Knoten 31 auf den Instrumentenkopf 19 gelangt und auf dessen Schräge 37 in Richtung des Stirnteils 35 abgleitet. Auf diesem Wege werden die freien Enden 27 von den Stiften 40 erfaßt und in den Schlitz 41 geleitet, während die zum Knoten 31 benachbarten Bereiche der Schlaufe 30 in den Längsschlitz 43 übergehen, was durch die zuvor beschriebene konische Ausbildung des Längsschlitz 43 begünstigt wird. Dabei ist es zweckmäßig, zur sicheren Plazierung der freien Enden 27 im Schlitz 41, die beiden freien Enden 27 etwas weiter um den Instrumentenkopf 19 herum zulegen. Die Bewegung des Instruments 10 erfolgt dabei also von der eigentlichen Nahtstelle weg, bis der Knoten 31 so weit auf der Schräge 37 abgeglitten ist, daß er in den Schenkel 36 der Ausnehmung 32 eingetreten, diesen durchlaufen hat und schließlich in die Bohrung 42 des zentralen Bereichs 29 der Profilierung gelangt ist. Dabei wird er ständig von den beiden freien Enden 27 mittig zur Schräge 37 gehalten. Etwa gleichzeitig erreichen die freien Enden 27 den Boden 39 des Freischliffs 38 von dem jeweiligen Stift 40 geführt und treten in die Ausnehmung 32 seitlich derselben ein. Ebenso gleiten währenddessen die benachbarten Schlaufenbereiche über den Längsschlitz 43 und den Spalt 44 in die Bohrung 42.

Mit dem Erreichen dieser Position beginnt eine Umkehr der Bewegung des Instruments 10, in dem es nun in Richtung der eigentlichen Nahtstelle verschoben wird. Dabei gleitet anfänglich der Knoten 31 in der Bohrung 42 entlang des Schenkels 33 und durch die Seitenführung 45 gehalten, bis er am Ende der Bohrung 42 angelangt ist. Anschließend setzt das Verschieben des derart sicher fixierten Knotens 31 ein. Dieses wird fortgesetzt bis der Knoten 31 in unmittelbarer Nähe des Zielpunkts angelangt ist, wobei das Verschieben wegen der Führung der freien Enden 27 und des gestrafften Garns sehr leicht erfolgen kann. Anschließend erfolgt das eigentliche Setzen des Knotens 31 präzise im Zielpunkt, indem mittels der Betätigungseinrichtung 20 die beiden Hälften 17, 18 des Instrumentenkopfs 19 derart zueinander um die Achse 16 verschwenkt werden, daß die von ihnen gehaltenen freien Enden 27 gespreizt werden. Durch dieses Spreizen vollzieht sich die abschließende Bewegung des Knotens 31 zum Zielpunkt sowie das Festziehen desselben. Letzteres kann zusätzlich dadurch begünstigt werden, daß der Zug auf die freien Enden etwas verstärkt wird, nachdem die Hälften 17, 18 zueinander gespreizt wurden. Wesentlich ist es, das die freien Enden 27 wie auch der Knoten 31 in jeder Phase sicher und separat mittels des Instrumentenkopfs 19 gehalten sind.

Nach dem Setzten des Knotens 31 werden die Hälften 17, 18 mittels der Betätigungseinrichtung 20 wieder aneinander angelegt, worauf das Instrument 10 ungehindert entfernt bzw. aus dem Trokar herausgezogen werden kann. Die freien Enden 27 gleiten dabei innerhalb des jeweiligen Spalts 44, der Ausnehmung 32 und der Bohrung 39 bis sie aus dem Instrumentenkopf heraustreten. Abschließend kann sodann an geeigneter Stelle das jeweilige freie Ende 27 gekürzt werden.

## Patentansprüche

1. Medizinisches Instrument (10) zum gezielten Setzen eines Knotens (31), der durch zwei freie Enden (27) eines chirurgischen Garns oder dgl. Nähmaterials unter Ausbildung einer endlosen Schlaufe (30) gebildet ist, mit einem Instrumentenkopf (19), der geteilt ist und zwei relativbewegliche Hälften (17; 18) aufweist, dadurch gekennzeichnet, daß der Instrumentenkopf (19) eine Profilierung mit mindestens einem peripheren Bereich (28) und einem zentralen Bereich (29) zur separaten Aufnahme, Führung und/oder Handhabung der freien Enden (27) des Garns und/oder Knotens (31) und benachbarter Abschnitte der Schlaufe (30) aufweist, beide Bereiche (28; 29) über eine Ausnehmung (32) miteinander verbunden sind und dem zentralen Bereich (29) eine koaxiale Bohrung (42) zugeordnet ist, die in die Ausnehmung (32) einmündet.

2. Medizinisches Instrument nach Anspruch 1, dadurch gekennzeichnet, daß der Instrumentenkopf (19) eine im wesentlichen zylindrische Gestalt aufweist und sein Durchmesser etwa dem einer angrenzenden Führungshülse (13) eines Grundkörpers entspricht.

3. Medizinisches Instrument nach Anspruch 1, dadurch gekennzeichnet, daß der periphere Bereich (28) insbesondere für die Handhabung der freien Enden (27) und der zentrale Bereich (29) zur Aufnahme und Führung des Knotens (31) und deren benachbarte Schlaufenbereiche vorgesehen sind.

4. Medizinisches Instrument nach Anspruch 3, dadurch gekennzeichnet, daß die Ausnehmung (32) den Instrumentenkopf (19) etwa senkrecht zu dessen Mittellinie (34) durchdringt und in Form eines spitzen Winkels ausgebildet ist.

5. Medizinisches Instrument nach einem oder mehreren der Ansprüche 3 und 4, dadurch gekennzeichnet, daß sich ein endlicher Schenkel (33) der Ausnehmung (32) zur Aufnahme der freien Enden (27) in Längsrichtung des Instrumentenkopfs (19) etwa entlang der Mittellinie (34) des letzteren und bis zu einem Stirnteil (35) des Instrumentenkopfs (19) erstreckt.

6. Medizinisches Instrument nach einem oder mehreren der Ansprüche 3 bis 5, dadurch gekennzeichnet, daß sich ein anderer Schenkel (36) der Ausnehmung (32) zur Aufnahme und Führung wenigstens des Knotens (31) von dem stirnteilseitigen Ende des Schenkels (33) und entgegengesetzt zum Stirnteil (35) spitzwinklig erstreckt und aus dem Instrumentenkopf (19) austritt.

7. Medizinisches Instrument nach einem oder mehreren der Ansprüche 5 und 6, dadurch gekennzeichnet, daß die Schenkel (33; 36) derart bemessen sind, daß der endliche Schenkel (33) von den freien Enden (27) des Garns und der anderen Schenkel (36) vom Knoten (31) passierbar sind.

8. Medizinisches Instrument nach einem oder mehreren der Ansprüche 1 bis 7, dadurch gekennzeichnet, daß der periphere Bereich (28) eine Schräge (37) zur Abstützung des Knotens (31) und der freien Enden (27) aufweist, die sich vom Außenumfang des Instrumentenkopfs (19) in Richtung des Stirnteils (35) und zur Mittellinie (34) des Instrumentenkopfs (19) erstreckt und etwa dort, wo die Schenkel (33; 36) ineinander übergehen, in der Ausnehmung (32) endet.

9. Medizinisches Instrument nach Anspruch 8, dadurch gekennzeichnet, daß die Schräge (37) umfänglich etwa über den halben Instrumentenkopf (19) annähernd gleichmäßig gewölbt ist.

10. Medizinisches Instrument nach einem oder mehreren der Ansprüche 8 und 9, dadurch gekennzeichnet, daß die Schräge (37) beiderseits mit einem Freischliff (38) versehen ist, der im Bereich seines Bodens (39) in den endlichen Schenkel (33) der Ausnehmung (32) einmündet.

11. Medizinisches Instrument nach Anspruch 10, dadurch gekennzeichnet, daß sich der Freischliff (38) vom Boden (39) zur Mittellinie (34) des Instrumentenkopfs (19) leicht geneigt und vom Stirnteil (35) wegweisend erstreckt.

12. Medizinisches Instrument nach einem oder mehreren der Ansprüche 10 und 11, dadurch gekennzeichnet, daß sich vom Boden (39) des Freischliffs (38) ein zu letzterem im wesentlichen parallel angeordneter Stift (40) erstreckt und ein einseitig offener Schlitz (41) zwischen dem Freischliff (38) und dem Stift (40) gebildet ist, in welchen ein freies Ende (27) des Garns einführ- und führbar ist.

13. Medizinisches Instrument nach Anspruch 12, dadurch gekennzeichnet, daß die Länge des Stifts (40) derart bemessen ist, daß er den Freischliff (38) und die Schräge (37) überragt, nicht aber in Radialrichtung des Instrumentenkopfs (19) über letzteren hinausragt.

14. Medizinisches Instrument nach einem oder mehreren der Ansprüche 2 bis 13, dadurch gekennzeichnet, daß mindestens der Knoten (31) im Stirnteil (35) des Instrumentenkopfs (19) mittels der koaxialen Bohrung (42) aufnehm- und führbar ist.

15. Medizinisches Instrument nach Anspruch 14, dadurch gekennzeichnet, daß der Durchmesser der Bohrung (42) größer als die lichte Weite des endlichen Schenkels (33) ist und die Bohrung (42) den Schenkel (33) in Axialrichtung des Instrumentenkopfs (19) vollständig durchdringt, derart, daß entlang der Bohrung (42) eine Seitenführung (45) des Knotens (31) gebildet ist.

16. Medizinisches Instrument nach einem oder mehreren der Ansprüche 14 und 15, dadurch gekennzeichnet, daß der Durchmesser der Bohrung (42) geringfügig größer als die Erstreckung des Knotens (31) in Radialrichtung des Instrumentenkopfs (19) ist.

17. Medizinisches Instrument nach einem oder mehreren der Ansprüche 14 bis 16, dadurch gekennzeichnet, daß der Bohrung (42) im Stirnteil (35) des Instrumentenkopfs (19) ein keilförmiger Längsschlitz (43) zugeordnet ist, der sich zur Bohrung (42) hin bis zu einem schmalen Spalt (44) für den Durchtritt der zum Knoten (31) benachbarten Schlaufenbereiche in die Bohrung (42) verjüngt.

18. Medizinisches Instrument nach Anspruch 17, dadurch gekennzeichnet, daß der Längsschlitz (43) in Längsrichtung des Instrumentenkopfs (19) zur Stirnfläche des Stirnteils (35) zusätzlich konisch aufgeweitet ist.

19. Medizinisches Instrument nach einem oder mehreren der Ansprüche 8 bis 18, dadurch gekennzeichnet, daß der Längsschlitz (43) und die Schräge (37) korrespondierend dem Umfang des Instrumentenkopfs (19) zugeordnet sind und in Längsrichtung symmetrisch im wesentlichen hintereinander liegen.

20. Medizinisches Instrument nach einem oder mehreren der Ansprüche 1 bis 19, dadurch gekennzeichnet, daß die Hälften (17; 18) zueinander schwenkbeweglich sind und den Hälften (17; 18) die Profilierung wie auch der Längsschlitz (43) symmetrisch zugeordnet sind.

21. Medizinisches Instrument nach einem oder mehreren der Ansprüche 1 bis 20, dadurch gekennzeichnet, daß die Hälften (17, 18) scherenförmig angeordnet und um eine einem Endbereich (15) der Führungshülse (13) zugeordnete Achse (16) derart schwenkbeweglich sind, daß die freien Enden (27) des Garns zueinander spreizbar sind.

## Claims

1. Medical instrument (10) for targeted knotting (31), which is formed by an endless loop (30) through two free ends (27) of a surgical yard or similar material with an instrument head (19) which is divided and has two relatively moveable halves (17; 18), characterized by, the instrument tip (19) having a profile with at least one peripheral area (28) and a central area (29) for separate uptake, guiding and/or handling of the free ends (27) of the yarn and/or knot (31) and the neighboring section of the loop (30), both areas (28; 29) are connected with an recess (32) and a coaxial hole (42) is placed in the central area, leading into the recess (32).

2. Medical instrument as in claim 1, characterized by the instrument tip (19) having an essentially cylindrical form and its diameter corresponding to a bordering sleeve (13) of a basic body.

3. Medical instrument in accordance with claim 1, characterized by the peripheral area (28) being intended especially for the handling of the free ends (27) and the central area (29) for holding and guiding the knot (31) and its neighboring loop areas.

4. Medical instrument in accordance with claim 3, characterized by the recess (32)penetrating the instrument tip (19) about vertically to its middle line (34) and shaped in the form of an acute angle.

5. Medical instrument in accordance with one or more of claims 3 and 4, characterized by a limited shank (33) of the recess (32) for holding the free ends (27) stretches lengthwise along the tip of the instrument (19) approximately along the middle line (34) of the latter and up to a head-end (35) of the tip of the instrument (19).

6. Medical instrument in accordance with one or more of claims 3 to 5, characterized by another shank (36) of the recess (32) for holding and guiding at least the knot (31) from the head-end of the shank (33) and stretches in the other direction in an acute angle to the head-end and leads out of the tip of the instrument (19).

7. Medical instrument in accordance with one or more of claims 5 and 6, characterized by the shanks (33; 36) being dimensioned in such a way that the free ends (27) of the yarn can pass through the limited shank (33) and the knot (31) can pass through the other shank (36).

8. Medical instrument in accordance with claims 1 to 7, characterized by the peripheral area (28) having a slope (37) to support the knot (31) and the free ends (27) which stretch from the exterior circumference of the tip of the instrument (19) in the direction of the head (35) and to the middle line (34) of the tip of the instrument (19) and ends in the recess (32) at about where the shanks (33;36) meet.

9. Medical instrument in accordance with claim 8, characterized by the slope (37) being about equally curved around about half of the tip of the instrument (19)

10. Medical instrument in accordance with one or more of claims 8 and 9, characterized by the slope having a cut (38) on each side that joins the recess (32) in the limited shank (33) in the area of its base (39),

11. Medical instrument in accordance with claim 10, characterized by the cut (38) from the base (39) to the middle line (34) of the tip of the instrument (19) being at a slight angle and stretching from the head (35).

12. Medical instrument in accordance with one or more of claims 10 and 11, characterized by a pin going parallel from the base (39) of the cut (38) forming an open slit (41) between the cut (38) and the pin (40) in which a free end (27) of the yarn can be placed and guided.

13. Medical instrument in accordance with claim 12, characterized by the length of the pin (40) being dimensioned so that it extends beyond the cut (38) and the slant (37), but does not extend beyond the latter in the radial direction of the tip of the instrument.

14. Medical instrument in accordance with one or more of claims 2 to 13, characterized by at least the knot (31) in the head (35) of the tip of the instrument (19) being picked up and guided by means of the coaxial hole (42).

15. Medical instrument in accordance with claim 14, characterized by the diameter of the hole (42) being larger than the inside diameter of the limited shank (33) and the hole (42) completely penetrates the shank (33) in the axial direction of the tip of the instrument (19) in such a way that a lateral guidance (45) of the knot (31) is formed along the hole (42).

16. Medical instrument in accordance with one or more of claims 14 and 15, characterized by the diameter of the hole (42) being slightly larger than the span of the knot (31) in the radial direction of the tip of the instrument (19).

17. Medical instrument in accordance with one or more of claims 14 to 16, characterized by the hole (42) in the head (35) of the tip of the instrument (19) having a wedge-shaped longitudinal slit (43) which is tapered to the hole (42) to a small gap (44) for the passage of the neighboring loop area of knot (31) into the hole (42).

18. Medical instrument in accordance with claim 17, characterized by the longitudinal slit (43) in the direction of the length of the tip of the instrument (19) is also tapered outward to the surface of the end of the head (35).

19. Medical instrument in accordance with one or more of claims 8 to 18, characterized by that the longitudinal slit (43) and the slant (37) correspond to the circumference of the tip of the instrument (19) and lengthwise are essentially symmetrically behind one another.

20. Medical instrument in accordance with one or more of claims 1 to 19, characterized by the halves (17; 18) swiveling toward one another and the profile and the longitudinal slit (43) of the halves (17; 18) are also symmetrical.

21. Medical instrument in accordance with one or more of claims 1 to 20, characterized by the halves (17, 18) being scissors like and swivel in such a manner around an axis (16) of the end area (15) of the guiding sleeve (13) that the free ends (27) of the yam can be spread to one another.

## Revendications

1. Instrument médical (10) destiné à positionner ponctuellement un noeud (31) formé par deux extrémités libres (27) d'un fil chirurgical ou d'un matériau à suture semblable en formant un noeud coulant (30) sans fin, muni d'une tête d'instrument (19) divisée et présentant deux moitiés (17;18) susceptibles de se déplacer l'une par rapport à l'autre, **caractérisé par le fait que** la tête d'instrument (19) présente un profilage muni au moins d'une zone périphérique (28) et d'une zone centrale (29) destinées à loger, guider et/ou manipuler les extrémités libres (27) du fil et/ou du noeud (31) et des segments adjacents du noeud coulant (30), que les deux zones (28;29) sont reliées ensemble par l'intermédiaire d'un creux (32) et qu'un perçage coaxial (42) débouchant dans le creux (32) est associé à la zone centrale (29).

2. Instrument médical selon la revendication 1, **caractérisé par le fait que** la tête d'instrument (19) présente une configuration sensiblement cylindrique et son diamètre correspond à peu près à celui d'un manchon de guidage (13) adjacent d'un corps de base.

3. Instrument médical selon la revendication 1, **caractérisé par le fait que** la zone périphérique (28) est prévue pour la manipulation des extrémités libres (27) et la zone centrale (29) pour loger et guider le noeud (31) et les zones du noeud coulant adjacentes.

4. Instrument médical selon la revendication 3, **caractérisé par le fait que** le creux (32) traverse la tête d'instrument (19) à peu près perpendiculairement par rapport à sa ligne médiane (34) et est développé en forme d'angle aigu.

5. Instrument médical selon l'une ou plusieurs des revendications 3 et 4, **caractérisé par le fait qu**'une aile finie (33) du creux (32) destinée à loger les extrémités libres (27) s'étire dans le sens longitudinal de la tête d'instrument (19) à peu près le long de la ligne médiane (34) de cette dernière et jusqu'à une partie frontale (35) de la tête d'instrument (19).

6. Instrument médical selon l'une ou plusieurs des revendications 3 à 5, **caractérisé par le fait qu**'une autre aile (36) du creux (32) destinée à loger et guider au moins le noeud (31) s'étire à angle aigu de l'extrémité de l'aile (33) située du côté de la partie frontale et à l'opposé de la partie frontale (35) et émerge hors de la tête d'instrument (19).

7. Instrument médical selon l'une ou plusieurs des revendications 5 et 6, **caractérisé par le fait que** les ailes (33;36) présente des dimensions telles que l'aile finie (33) est susceptible d'être traversée par les extrémités libres du fil et l'autre aile (36) est susceptible d'être traversée par le noeud (31).

8. Instrument médical selon l'une ou plusieurs des revendications 1 à 7, **caractérisé par le fait que** la zone périphérique (28) présente une partie inclinée (37) destinée à supporter le noeud (31) et les extrémités libres (27) qui s'étire du périmètre extérieur de la tête d'instrument (19) en direction de la partie frontale (35) et jusqu'à la ligne médiane (34) de la tête d'instrument (19) et se termine dans le creux (32) à peu près à l'endroit où les ailes (33;36) s'interpénètrent.

9. Instrument médical selon la revendication 8, **caractérisé par le fait que** la partie inclinée (37) est cintrée à peu près régulièrement sur son périmètre à peu près sur la moitié de la tête d'instrument (19).

10. Instrument médical selon l'une ou plusieurs des revendications 8 et 9, **caractérisé par le fait que** la partie inclinée (37) est pourvue des deux côtés d'une section polie libre (38) qui débouche dans la zone de son fond (39) dans les ailes finies (33) du creux (32).

11. Instrument médical selon la revendication 10, **caractérisé par le fait que** la section polie libre (38) s'étire du fond (39) jusqu'à la ligne médiane (34) de la tête d'instrument (19) en présentant une légère déclivité et en s'écartant de la partie frontale (35).

12. Instrument médical selon l'une ou plusieurs des revendications 10 et 11, **caractérisé par le fait qu**'une broche (40) disposée sensiblement parallèlement au fond (39) de la section polie libre (38) s'étire à partir de celui-ci et qu'une fente ouverte d'un côté (41) est formée entre la section polie libre (38) et la broche (40), fente dans laquelle une extrémité libre (27) du fil est susceptible d'être introduite et guidée.

13. Instrument médical selon la revendication 12, **caractérisé par le fait que** la longueur de la broche (40) présente des dimensions telles que celle-ci dépasse de la section polie libre (38) et de la partie inclinée (37), mais n'émerge pas hors de la tête d'instrument (19) dans le sens radial de celle-ci.

14. Instrument médical selon l'une ou plusieurs des revendications 2 à 13, **caractérisé par le fait qu**'au moins le noeud (31) est susceptible d'être logé et guidé dans la partie frontale (35) de la tête d'instrument (19) au moyen du perçage coaxial (42).

15. Instrument médical selon la revendication 14, **caractérisé par le fait que** le diamètre du perçage (42) est supérieur à l'ouverture de l'aile fini (33) et que le perçage (42) traverse complètement l'aile (33) dans le sens axial de la tête d'instrument (19) de telle façon qu'un guidage latéral (45) du noeud (31) est formé le long du perçage (42).

16. Instrument médical selon l'une ou plusieurs des revendications 14 et 15, **caractérisé par le fait que** le diamètre du perçage (42) est à peu près supérieur à l'étirement du noeud (31) dans le sens radial de la tête d'instrument (19).

17. Instrument médical selon l'une ou plusieurs des revendications 14 à 16, **caractérisé par le fait qu**'un trou oblong (43) cunéiforme est associé au perçage (42) dans la partie frontale (35) de la tête d'instrument (19), trou se rétrécissant jusqu'au perçage (42) pour former une étroite fente (44) pour le passage dans le perçage (42) des zones du noeud coulant adjacentes au noeud (31).

18. Instrument médical selon la revendication 17, **caractérisé par le fait que** le trou oblong (43) est en plus élargi en forme de cône dans le sens longitudinal de la tête d'instrument (19) par rapport à la surface frontale de la partie frontale (35).

19. Instrument médical selon l'une ou plusieurs des revendications 8 à 18, **caractérisé par le fait que** le trou oblong (43) et la partie inclinée sont associés de façon correspondante au périmètre de la tête d'instrument (19) et qu'ils se trouvent symétriquement sensiblement l'un derrière l'autre dans le sens longitudinal.

20. Instrument médical selon l'une ou plusieurs des revendications 1 à 19, **caractérisé par le fait que** les moitiés (17;18) sont susceptibles de pivoter l'une par rapport à l'autre et le profilage comme le trou oblong (43) sont associés symétriquement aux moitiés (17;18).

21. Instrument médical selon l'une ou plusieurs des revendications 1 à 20, **caractérisé par le fait que** les moitiés (17;18) sont disposées à la façon de ciseaux et sont susceptibles de pivoter autour d'un axe (16) associé à une zone terminale (15) du manchon de guidage (13) de telle façon que les extrémités libres (27) du fil sont susceptibles de s'écarter l'une de l'autre.
